# EUROPEAN PATENT SPECIFICATION

(11) **EP 2 994 190 B1**
(45) Date of publication and mention of the grant of the patent: **28.02.2018**
(21) Application number: 14726208.3
(22) Date of filing: 08.05.2014
(51) Int. Cl.: A61N 1/39, A61N 1/04

(54) **DEFIBRILLATOR ELECTRODE HAVING ADULT AND PEDIATRIC GRAPHICS**
DEFIBRILLATIONSELEKTRODE MIT ERWACHSENEN- UND PÄDIATRISCHER GRAFIK
ÉLECTRODE DE DÉFIBRILLATEUR AYANT DES GRAPHIQUES POUR ADULTES ET PÉDIATRIQUES

(30) Priority: 09.05.2013 US 201361821269 P
(43) Date of publication of application: 16.03.2016
(73) Proprietor: Koninklijke Philips N.V., 5656 AE Eindhoven (NL)
(72) Inventor: PIAZZA, Aaron James, 5656 AE Eindhoven (NL)
(74) Representative: Coops, Peter
(86) International application number: PCT/IB2014/061287
(87) International publication number: WO 2014/181277

(56) References cited:
- US-A1- 2002 082 672
- US-A1- 2003 199 929
- US-A1- 2012 253 162
- US-A9- 2010 063 558

## Description

The present invention relates generally to an electrotherapy apparatus for delivering a series of shocks to a patient's heart. More particularly, this invention pertains to an improved Automated External Defibrillator (AED) and electrode set that is suitable for defibrillating patients of all ages.

Sudden Cardiac Arrest (SCA) is one of the leading causes of death in the industrialized world, and typically results from an arrhythmia condition known as Ventricular Fibrillation (VF), during which a patient's heart muscle exhibits extremely rapid, uncoordinated contractions that render the heart incapable of circulating blood. Statistically, after the first four minutes, the patient's chance of survival decreases by approximately 10% during each subsequent minute they fail to receive treatment.

A generally effective treatment for VF is electrical defibrillation, in which a defibrillator delivers an electrical pulse or shock to the patient's heart. Because the onset of VF is generally an unpredictable event, the likelihood that a victim will survive rises dramatically if defibrillation equipment is nearby. As a result, medical equipment manufacturers have developed Automated External Defibrillators (AEDs) that minimally trained personnel may employ to perform electrical defibrillation when emergency situations arise. AEDs may be found in non-medical settings such as residences, public buildings, businesses, private vehicles, public transportation vehicles, and airplanes, for example.

To increase a patient's chances of survival, AED operators generally must perform quickly and accurately in life-threatening situations. Hence, AEDs are typically designed to be simple and intuitive to use. AEDs often automate many of the steps associated with operating external defibrillation equipment, and minimize the number of decisions the operator must make. An AED may provide voice instructions or commands to guide the operator through application of the device. An AED may also analyze a patient's heart rhythm to determine when administration of an electrical shock to the patient may be appropriate. If a shock is warranted, the AED facilitates delivery of a defibrillation waveform at a particular energy level.

Some defibrillators have included graphics on the electrode pads that are applied to the patient for delivering electrotherapy. One co-assigned patent, U.S. Patent 5,951,598 entitled "Electrode system", describes, e.g., a printed graphic on each electrode which shows an image of at least a portion of an adult human body, further having an image of the proper position of the electrode pad on the body image. In addition, US 2002/082672 A1 and US 2010/063558 A9 also describe defibrillation electrodes with graphics showing a portion of an adult human body and the location as to where the electrode is to be placed.

The large majority of VF situations involve adult patients, as VF tends to be a relatively rare condition in children and/or infants. Nonetheless, recent evidence suggests that pediatric VF occurs with sufficient frequency to be of concern. AEDs, however, are generally designed for use on/with adults. Further, in the past, pediatric application of AEDs had generally been limited by a lack of data characterizing pediatric Electrocardiogram (ECG) rhythms, which generally cast doubt upon the effectiveness of EGG detection algorithms that an AED may employ in pediatric situations.

Additionally, energy delivery recommendations for children are dependent upon body mass, whereas such recommendations for adults generally are not. Presently, the recognized treatment for pediatric VF in children less than 8 years of age is manual defibrillation in which delivered energies are proportional to the patient's body weight (e.g., 1 Joule per kilogram of body weight, increasing to 2 Joules per kilogram if necessary). Incorporating controls to facilitate detailed energy adjustments in accordance with body mass or weight would generally add extra complexity and cost to an AED design. Moreover, providing such controls could/would undesirably complicate the decisions operators must make during time-critical situations, even when treating adults, thereby providing more opportunity for treatment to fail and generally decreasing the patient's chance of the survival.

Some defibrillators, such as disclosed in US2003/0055459 A1, may include an adult/pediatric mode control or switch. Based upon a setting of the adult/pediatric switch, the AED may perform an adult defibrillation sequence or a pediatric defibrillation sequence upon a patient. An adult defibrillation sequence may involve the delivery of one or more defibrillator shocks characterized by an energy appropriate for adults. For example, an adult defibrillation sequence may comprise the delivery of one or more 150 Joule biphasic waveforms to the patient, whereas a pediatric defibrillation sequence may involve the delivery of one or more defibrillator shocks characterized by an energy appropriate for children who are or seem to be less than eight years of age. For example, a pediatric defibrillation sequence may comprise the delivery of one or more 50 Joule biphasic defibrillator shocks to the patient.

Furthermore, a defibrillator such as described above may employ a universal electrode suitable for use on all patients that is smaller than a conventional adult electrode, yet larger than a conventional pediatric electrode. For example, such a defibrillator and electrode are described in co-assigned U.S. Patent 7,062,321 entitled "Method and apparatus for defibrillating patients of all ages", the entire disclosure of which is incorporated herein by reference.

Graphics that appear on existing electrodes, however, generally restrict their placement guidance to either adult or pediatric patients, e.g., in order to minimize confusion during the much-more-common adult cardiac rescues. But such graphics simplify the electrode placement only if used in situations matching the patient, and can cause/induce confusion or delay in situations where the graphic type and the patient differ from one another. For example, if a universal electrode is used on a pediatric patient, but has only adult placement markings, the rescuer must recollect prior training or take the time to look up the proper placement, e.g., in an instruction manual to know that the preferred placement generally is anterior-posterior instead of the illustrated apex-sternum adult placement. This problem can be especially exacerbated in defibrillators having a mode switch.

What is needed therefore is an improved user guidance illustration for a universal electrode, which provides electrode placement guidance for patients of all ages.

In accordance with the objectives of the present invention, the inventors have invented and developed, e.g., a graphic illustration which intuitively guides the user to correct placement in both the adult and pediatric cardiac rescues. By inventively arranging both adult and pediatric graphics on each of the electrodes, guidance is provided for both types of rescue. Furthermore, by inventive sizing, orientation, and coloring of the graphics, exemplary embodiments of the present invention can provide the appropriate amount of information and guidance while avoiding confusion, distraction and time delays that could otherwise be caused.

An exemplary embodiment of an electrode for a defibrillator is described comprising a lead wire, a substrate, and a conductive surface disposed on one side of the substrate and electrically connected to the lead wire. The other graphics side of each electrode includes a printed graphic showing the proper positioning of the electrode on a patient torso, wherein the graphic includes both a figure of an adult body and of a pediatric body.

In accordance with an aspect of the invention, an exemplary embodiment of an external defibrillator is described comprising a high voltage energy source structured, configured and operable to deliver electrotherapy, an electrode connector electrically connected to the high voltage energy source, a controller, and a mode switch operable to switch the controller from an adult defibrillation mode to a pediatric defibrillation mode. In accordance with exemplary embodiments of the present invention, the defibrillator can also include an electrode storage device having a release liner surface, and first and second universal electrode pads disposed on the release liner surface, each electrode pad having a lead wire connected to the electrode connector, a printable surface, and a graphic indication disposed on the printable surface of the proper placement of the respective electrode pad on both of an adult human body and a pediatric human body.

In accordance with another aspect of the invention, an exemplary embodiment of a medical electrode system is described comprising first and second electrode pads having a lead wire electrically connected to each electrode pad. Each of the first and second electrode pads comprises a patient-facing side adapted to be placed on a patient and a graphics side adapted to be visible when the patient-facing side is placed on a patient. The graphics side of the first electrode pad comprises, e.g., a first image of at least a portion of an adult human body showing a proper adult placement of the first electrode pad, and a second image of at least a portion of a pediatric human body showing a proper pediatric placement of the first electrode pad. The graphics side of the second electrode pad comprises, e.g., a third image of at least a portion of an adult human body showing a second proper adult placement of the second electrode pad, and a fourth image of at least a portion of a pediatric human body showing a second proper pediatric placement of the second electrode pad.

The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims and equivalents thereof.
FIGUREs 1a and 1b illustrate a medical electrode system according to an exemplary embodiment of the present invention.
FIGURE 2 illustrates a graphic indicator on one medical electrode pad according to an exemplary embodiment of the present invention.
FIGURE 3 illustrates a graphic indicator on another medical electrode pad according to an exemplary embodiment of the present invention.
FIGUREs 4a and 4b illustrate a defibrillator including electrodes according to an exemplary embodiment of the present invention.
FIGURE 5 illustrates an exemplary embodiment of the present invention in use during a cardiac rescue, in accordance with the present disclosure.

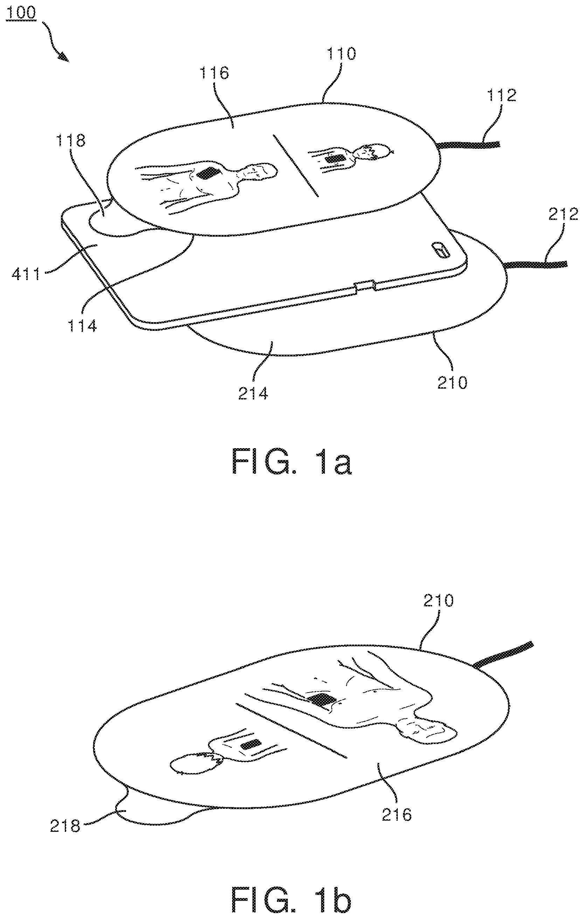

Referring first to FIGURE 1a, a medical electrode system 100 is illustrated according to an exemplary embodiment of the present invention. The arrangement of the electrode system 100 can be similar in some respects to the electrode illustrates in co-assigned U.S. Patent Publication 2007/0203558 entitled "Self-storing Medical Electrodes". Shown in Figure 1a are two electrode pads 110, 210 disposed on a central release liner sheet 411, which is hereafter referred to as a hardliner.

Hardliner 411 is a substantially rigid sheet having a release liner surface on both sides. The two electrode pads 110, 210 are disposed on either side of the hardliner 411 with the patient-facing side of each pad in a face-to-face relationship.

Each electrode pad 110, 210 includes a respective lead wire 112, 212 which is electrically attached to a conductive surface disposed on a substrate, the conductive surface being on a patient-facing side 114 or 214. The conductive surface may include a conductive gel for adhering to the patient's skin, and for conveying defibrillation energy from the electrode to the patient. Each electrode patient-facing side is disposed on a release surface on each side of hardliner 411 for storage. In order to facilitate the deployment of the electrode pads 110,210 from hardliner 411, each electrode may include a respective peel tab 118, 218.

As shown in Figure 1a, the other side of the first electrode pad 110 substrate, referred to as the graphics side 116, includes visual indications which are intended to guide the user to place the electrode properly on the patient. Graphics side 116 is preferably a printable surface. Similarly, as shown in FIGURE 1b, the second electrode pad 210 patient-facing graphics side 216 includes visual indications which are intended to guide the user to place the second electrode properly on the patient.

By placing the graphic on the electrode itself, the rescuer need only refer to the electrode in hand during the placement procedure. Thus, the rescuer does not have to look back and forth to other instructions during the rescue.

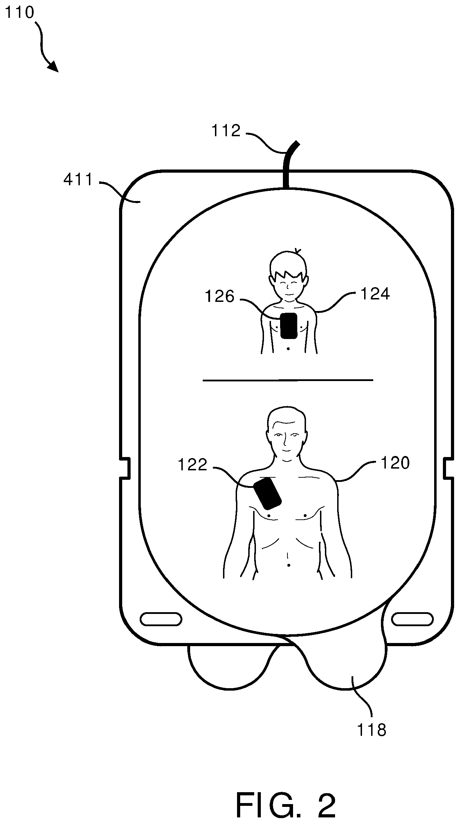

FIGURE 2 illustrates a more detailed view of the graphics side 116 of first electrode pad 110, as it resides on a hardliner 411. Graphics side 116 includes a first image 120 of at least a portion of an adult human body, on which is shown a proper adult placement 122 for that electrode pad 110. In this embodiment, the first electrode pad 110 is shown in a sternum position on the first image 120.

Also included on graphics side 116 is a second image 124 of at least a portion of a pediatric human body showing a proper pediatric placement 126 of the first electrode pad 110. In this embodiment, the first electrode pad 110 would be shown in an anterior position on the second image 124. Preferably, the relative size of the pediatric human body image 124 to the adult human body image 126 is in proportion to the actual size of an adult and a pediatric human, in order to further clarify the proper placement, and to emphasize the electrode placement in the more-common adult cardiac rescue.

The images of the adult human body and the pediatric human body may be two dimensional or three dimensional renderings. Additional, the images may include anatomical markers that assist the user in proper placement of the electrode pad 110. For example, depictions of the rib line, the clavicle bone (or collar bone), or the definition of the pectoral muscles may be shown. Additionally, the images of the human body preferably appear as the mirror image of the defibrillator operator, which corresponds to the positioning of the patient when the electrode pads are applied by the operator during treatment.

The adult placement 122 and/or pediatric placement 126 may be of a distinguishing color, pattern, or contrast with the underlying figure graphic 120, 124. The relative size of the placement graphics may be varied somewhat, and may be chosen to show a precise desired position, an acceptable zone of placement on the patient body, or an area somewhere in between.

Although not shown here, arrows, circles, or other features intended to direct the rescuer's attention to the placement instruction may be provided. Also not shown is an additional optional graphic which shows the proper placement of the other, second, electrode placement on the first image 120. This optional graphic would preferably be printed in a less conspicuous emphasis, such as in a lighter weight or a dashed line.

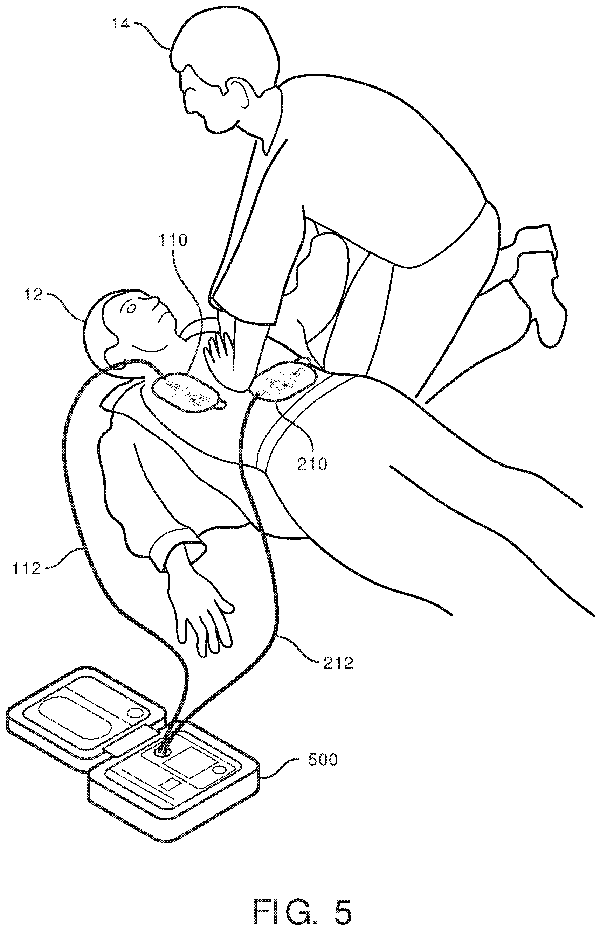

The electrode pad 110 of FIGURE 2 shows both of the first image 120 and the second image 124 to be oriented such that each image appears upright with a long dimension of the pad 110 to the left, i.e. in a portrait orientation. Thus, with reference to FIGURE 5, each graphic image is oriented on the printable surface in the same orientation as to an intended placement of the respective electrode on the patient 12, such that the rescuer 14 is assured that the electrode is correctly positioned without the need for any other reference. Thus, the rescuer 14 can direct his concentration to the time-critical step of applying the defibrillation electrodes. This allows the connected defibrillator 500 to deliver electrotherapy via the electrodes if necessary.

FIGURE 2 also illustrates the orientation of the lead wire 112 with respect to the electrode pad 110 and graphics. As shown, lead wire 112 is electrically connected to the first electrode pad 110 on a short dimension of the pad, and in particular above the heads of the adult and pediatric body images 120, 124. In reference again to FIGURE 5, it can be seen that this lead wire disposition enables a "wires out" deployment when the electrode pad 110 is placed as intended on the adult patient 12. In the "wires out" orientation, the lead wire extends away from the center of the torso for unobstructed access to the chest for CPR compressions and such.

Electrode pad 110 may further comprise a peel tab 118 disposed at one end of the electrode. Preferably, peel tab 118 has a distinguishing color or marking for guiding the user to grasp the peel tab for deployment and positioning the electrode 110 to the patient.

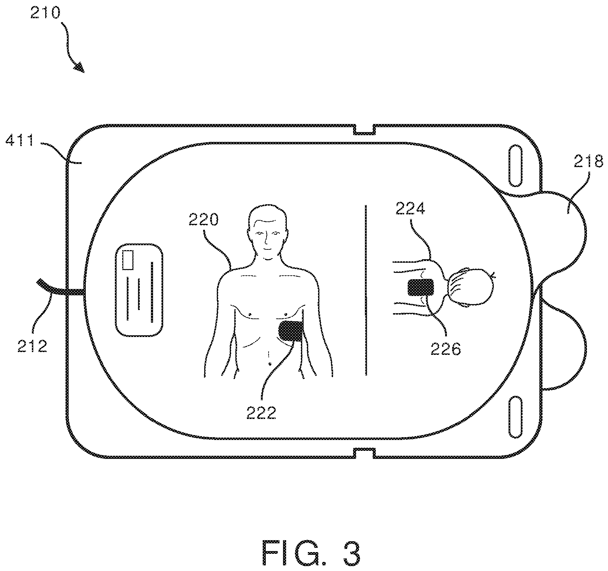

FIGURE 3 illustrates a more detailed view of the graphics side 216 of second electrode pad 210 as it resides on a hardliner 411. Graphics side 216 includes a third image 220 of at least a portion of an adult human body, on which is shown a proper adult placement 222 for that electrode pad 210. In this embodiment, the second electrode pad 210 is shown in an apex position on the third image 220.

Also included on graphics side 216 is a fourth image 224 of at least a portion of a pediatric human body showing a proper pediatric placement 226 of the second electrode pad 210. In this embodiment, the second electrode pad 210 would be shown in a posterior position on the fourth image 224. Preferably, the relative size of the pediatric human body image 224 to the adult human body image 226 is in proportion to the actual size of an adult and a pediatric human, in order to further clarify the proper placement, and to emphasize the electrode placement in the more-common adult cardiac rescue.

The images of the adult human body and the pediatric human body may be two dimensional or three dimensional renderings. Additional, the images may include anatomical markers that assist the user in proper placement of the electrode pad 210. For example, depictions of the rib line, the clavicle bone (or collar bone), or the definition of the pectoral muscles may be shown. Additionally, the images of the human body preferably appear as the mirror image of the defibrillator operator, which corresponds to the positioning of the patient when the electrode pads are applied by the operator during treatment.

The adult placement 222 and/or pediatric placement 226 may be of a distinguishing color, pattern, or contrast with the underlying figure graphic 220, 224. The relative size of the placement graphics may be varied somewhat, and may be chosen to show a precise desired position, an acceptable zone of placement on the patient body, or an area somewhere in between.

Although not shown here, arrows, circles, or other features intended to direct the rescuer's attention to the placement instruction may be provided. Also not shown is an additional optional graphic which shows the proper placement of the other, first, electrode placement on the third image 220. This optional graphic would preferably be printed in a less conspicuous emphasis, such as in a lighter weight or a dashed line.

The electrode pad 210 of FIGURE 3 shows the third image 220 to be oriented such that the adult body appears upside down with a short dimension of the pad 210 to the left, i.e. in a landscape orientation. Thus, with reference to FIGURE 5, the adult body graphic image is oriented on the printable surface in the same orientation as to an intended placement of the respective electrode on the adult patient 12, such that the rescuer 14 is assured that the electrode is correctly positioned without the need for any other reference. Thus, the rescuer 14 can direct his concentration to the time-critical step of applying the defibrillation electrodes.

The fourth image 224 of the pediatric patient is oriented on the electrode pad 210 rotated about a quarter turn clockwise from the third image 220 orientation. This orientation corresponds to the same orientation as to an intended placement of the pad 220 on the actual pediatric patient, i.e. vertical orientation on the posterior of the pediatric patient. Again, this orientation assists the rescuer 14 in quickly and accurately placing the electrode 210 on the pediatric patient.

FIGURE 3 also illustrates a preferred the orientation of the lead wire 212 with respect to the electrode pad 210 and graphics. As shown, lead wire 212 is electrically connected to the second electrode pad 210 on a short dimension of the pad, and in particular to the left side of the adult body image 220. In reference again to FIGURE 5, it can be seen that this lead wire disposition leads to a "wires in" deployment when the electrode pad 210 is placed as intended on the adult patient 12. The inventor has discovered that this disposition for the electrode when mounted at the apex position keeps the lead wire clear of the chest center in most situations.

It is contemplated, however, that a "wires out" orientation, wherein the lead wire extends away from the center of the torso for unobstructed access to the chest for CPR compressions and such, could alternatively be adopted for the second electrode pad 210. This arrangement can be accomplished by inverting the adult human body image 220 on the second electrode 210.

Electrode pad 210 may further comprise a peel tab 218 disposed at one end of the electrode. Preferably, peel tab 218 has a distinguishing color or marking for guiding the user to grasp the peel tab for deployment and positioning the electrode 210 to the patient. As can be seen in FIGURES 2 and 3, the peel tabs 118, 218 are preferably oriented at the same end of the hardliner 411, but offset somewhat for ease of use.

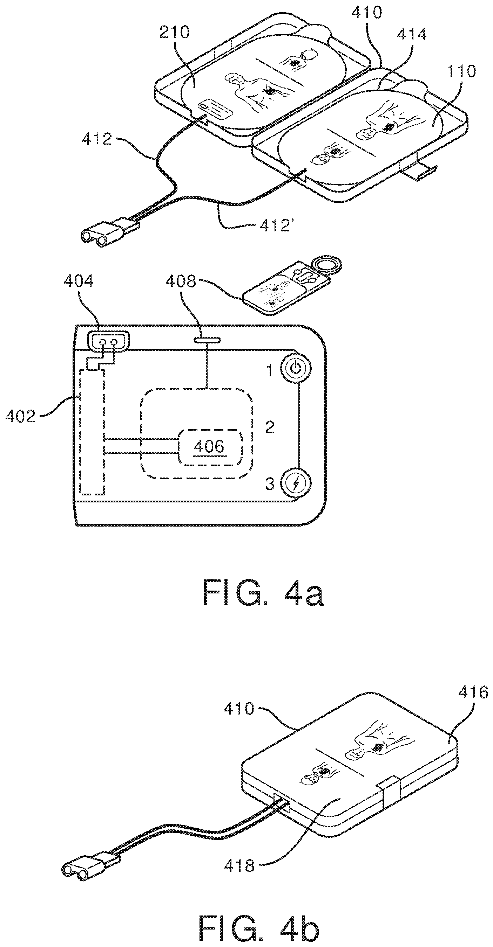

FIGUREs 4a and 4b illustrate another embodiment of the invention utilizing the electrode system 100 as previously described with a portable external defibrillator 400. In this embodiment, a clamshell case 410 is employed to store electrodes 110, 210 prior to use as an alternative to hardliner 411. The clamshell case 410 has a release liner surface 414 on the interior to which the patient-facing side of each of the electrode pads is attached.

FIGURE 4b illustrates the clamshell case 410 in the closed position, enclosing the electrodes inside. In order to further enhance the placement guidance graphics, it is preferred that at least one of the graphics from the pad graphics side 116, 216 is reproduced on a clamshell graphic 418 disposed on the clamshell exterior surface 416. The clamshell graphic 418 is preferably oriented the same as the graphic on the enclosed electrode pad. Thus placement guidance to the rescuer 14 begins even before the clamshell case 410 is opened.

Returning to FIGURE 4a, a defibrillator 400 is shown having a high voltage energy source 402 which delivers electrotherapy to the patient 12 via an electrode connector 404, lead wires 412,412' and electrodes 110, 210 to complete a circuit. First and second universal electrode pads 110, 210 are shown in the stored position. It is preferred that the electrodes 110, 210 are pre-connected to the electrode connector 404 during storage to facilitate even more rapid deployment in an emergency.

A controller 406 controls the electrotherapy circuit, and in particular adjusts the electrotherapy parameters via an adult/pediatric mode switch 408. The mode switch is operable to cause the controller to switch the defibrillator between an adult defibrillator mode and a pediatric mode. The mode switch 408 in this embodiment is a key, known as a pedi-key, which when inserted into a corresponding slot in defibrillator 400 by the user, causes controller 406 to switch the device into a pediatric operating mode. The pediatric operating mode generally entails delivery of a reduced-energy defibrillation shock.

Several variations within the scope of the afore-described invention as defined by the appended claims will readily occur to those skilled in the art. For instance, the hardliner may be substituted for the clamshell storage in the defibrillator system. The graphics may also vary in design elements somewhat, as long as they obtain the objects of the invention as defined by the appended claims.

### Table of Elements

- 12: patient
- 14: rescuer
- 100: medical electrode system
- 110: first electrode pad
- 112: first lead wire
- 114: first electrode pad patient-facing side
- 116: first electrode pad graphics side
- 118: first electrode pad peel tab
- 120: first image of adult
- 122: adult position image for first electrode pad
- 124: second image of pediatric patient
- 126: pediatric position image for first electrode pad
- 210: second electrode pad
- 212: second lead wire
- 214: second electrode pad patient-facing side
- 216: second electrode pad graphics side
- 218: second electrode pad peel tab
- 220: third image of adult
- 222: adult position image forsecibd electrode pad
- 224: fourth image of pediatric patient
- 226: pediatric position image for second electrode pad
- 400: external defibrillator
- 402: high voltage energy source
- 404: electrode connector
- 406: controller
- 408: mode switch
- 410: clamshell case
- 411: hardliner
- 412: lead wire
- 412': lead wire
- 414: clamshell release liner surface
- 416: clamshell exterior surface
- 418: clamshell graphic
- 500: defibrillator

## Claims

1. A medical electrode system (100) comprising:
- first and second electrode pads (110, 210); and
- a lead wire (112, 212) electrically connected to each electrode pad;
wherein each of said first and second electrode pads comprises a patient-facing side adapted to be placed on a patient and a graphics side (116, 216);
and further wherein the graphics side of the first electrode pad comprises:
- a first image of at least a portion of an adult human body (120) showing a proper adult placement of the first electrode pad, and
- a second image of at least a portion of a pediatric human body (124) showing a proper pediatric placement of the first electrode pad;
and further wherein the graphics side of the second electrode pad comprises:
- a third image of at least a portion of an adult human body (220) showing a second proper adult placement of the second electrode pad, and
- a fourth image of at least a portion of a pediatric human body (224) showing a second proper pediatric placement of the second electrode pad.

2. The medical electrode system of Claim 1
wherein each of the proper adult placement of the first electrode pad in the first image, the proper pediatric placement of the first electrode pad in the second image, the second proper adult placement of the second electrode pad in the third image, and the second proper pediatric placement of the second electrode pad in the fourth image have a distinguishing printing.

3. The medical electrode system of Claim 2 wherein the first image further comprises an image of the second proper adult placement of the second electrode pad and the third image further comprises an image of the proper adult placement of the first electrode pad.

4. The medical electrode system of Claim 1 wherein the proper pediatric placement of the first electrode pad is an anterior position on the second image, and wherein the second proper pediatric placement of the second electrode pad is a posterior position on the fourth image.

5. The medical electrode system of Claim 4, wherein each of the images of at least a portion of the pediatric human body is sized in proportion to each of the images of at least a portion of the adult human body relative to the actual size of an adult and a pediatric human.

6. The medical electrode system of Claim 1, wherein each of the first image, second image, third image and fourth image is oriented on the graphics side of the respective electrode pad such that the image body is aligned with the patient when the respective electrode pad is adhered in the correct position on the patient.

7. The medical electrode system of Claim 6, wherein a long dimension of the first electrode pad is oriented to the left of the first image and the second image, and further wherein a short dimension of the second electrode pad is oriented to the left of the third image, and further wherein a long dimension of the second electrode pad is oriented to the left of the fourth image when each of the first, second, third and fourth images are viewed in an upright position.

8. The medical electrode system of Claim 7, wherein the first electrode pad lead wire is connected to the first electrode pad at a short dimension edge above the first and second images as viewed in the upright position.

9. The medical electrode system of Claim 1 further comprising a peel tab disposed at one end of each electrode pad, and further wherein each peel tab has a distinguishing color.

10. The medical electrode system of Claim 1, further comprising a clamshell case with an exterior surface and a release liner disposed on an interior surface of the case, wherein the patient-facing side of each of the first and second electrode pads is attached to the release liner,
and further wherein each of the first image, second image, third image, and fourth image is disposed on the exterior surface in the same orientation as the respective image on the attached electrode pads.

11. An external defibrillator (400) comprising:
- a high voltage energy source (402) operable to deliver electrotherapy;
- an electrode connector (404) electrically connected to the high voltage energy source;
- a controller (406);
- a mode switch (408) operable to switch the controller from an adult defibrillation mode to a pediatric defibrillation mode;
- an electrode storage device (410, 411) having a release liner surface; and
- a medical electrode system (100) of Claim 1;
wherein the first and second electrode pads are first and second universal electrode pads (110, 210) disposed on the release liner surface;
wherein the lead wire (412, 412') electrically connected to each electrode pad is further connected to the electrode connector;
wherein the graphics side (116, 216) of each electrode pad is a printable surface; and
wherein the first and second images are a graphic indication disposed on the printable surface of the first electrode pad, and the third and fourth images are a graphic indication disposed on the printable surface of the second electrode pad.

12. The external defibrillator of Claim 11, wherein the electrode storage device is either:
- a clamshell having an interior release liner surface, wherein at least one of the graphic indications is reproduced on an exterior surface of the clamshell; or
- a sheet having the release liner surface on both sides, wherein each of the first and second universal electrode pads are disposed in a face to face relationship on opposite sides of the sheet.

13. The external defibrillator of Claim 11, wherein the mode switch is disposed as a key which is inserted into a corresponding slot in the defibrillator.

14. The external defibrillator of Claim 11, wherein each graphic indication is oriented on the printable surface in the same orientation as to an intended placement of the respective electrode on the adult and pediatric patient.

15. The external defibrillator of Claim 14, wherein the first universal electrode pad is disposed for attaching to the sternum position on an adult patient, and further wherein the first universal electrode pad lead wire is attached to the first universal electrode pad at a point that is away from the center of the adult patient torso when the first universal electrode pad is placed as intended on the adult patient.

## Patentansprüche

1. Medizinisches Elektrodensystem (100), umfassend:
- erste und zweite Elektrodenpads (110, 210); und
- ein Ableitungskabel (112, 212), das elektrisch mit jedem Elektrodenpad verbunden ist;
wobei sowohl das erste als auch das zweite genannte Elektrodenpad eine dem Patienten zugewandte Seite, die dafür ausgelegt ist, auf dem Patienten platziert zu werden, und eine Grafikseite (116, 216) umfasst;
und wobei weiterhin die Grafikseite des ersten Elektrodenpads Folgendes umfasst:
- ein erstes Bild von mindestens einem Teil des Körpers eines erwachsenen Menschen (120), das eine korrekte Platzierung des ersten Elektrodenpads bei dem Erwachsenen zeigt, und
- ein zweites Bild von mindestens einem Teil des Körpers eines Kindes (124), das eine korrekte Platzierung des ersten Elektrodenpads bei dem Kind zeigt;
und wobei weiterhin die Grafikseite des zweiten Elektrodenpads Folgendes umfasst:
- ein drittes Bild von mindestens einem Teil des Körpers eines erwachsenen Menschen (220), das eine korrekte Platzierung des zweiten Elektrodenpads bei dem Erwachsenen zeigt, und
- ein viertes Bild von mindestens einem Teil des Körpers eines Kindes (224), das eine korrekte Platzierung des zweiten Elektrodenpads bei dem Kind zeigt.

2. Medizinisches Elektrodensystem nach Anspruch 1,
wobei die korrekte Platzierung des ersten Elektrodenpads bei dem Erwachsenen in dem ersten Bild, die korrekte Platzierung des ersten Elektrodenpads bei dem Kind in dem zweiten Bild, die zweite korrekte Platzierung des zweiten Elektrodenpads bei dem Erwachsenen in dem dritten Bild und die zweite korrekte Platzierung des zweiten Elektrodenpads bei dem Kind in dem vierten Bild jeweils ein unterschiedliches Druckbild aufweisen.

3. Medizinisches Elektrodensystem nach Anspruch 2, wobei das erste Bild weiterhin ein Bild der zweiten korrekten Platzierung des zweiten Elektrodenpads bei dem Erwachsenen umfasst und das dritte Bild weiterhin ein Bild der korrekten Platzierung des ersten Elektrodenpads bei dem Erwachsenen umfasst.

4. Medizinisches Elektrodensystem nach Anspruch 1, wobei die korrekte Platzierung des ersten Elektrodenpads bei einem Kind auf dem zweiten Bild eine anteriore Position ist, und wobei die zweite korrekte Platzierung des zweiten Elektrodenpads bei dem Kind auf dem vierten Bild eine posteriore Position ist.

5. Medizinisches Elektrodensystem nach Anspruch 4, wobei jedes der Bilder des mindestens einen Teils des Körpers eines Kindes verhältnismäßig zu jedem der Bilder des mindestens einen Teils des Körpers eines Erwachsenen entsprechend der tatsächlichen Größe eines Erwachsenen und eines Kindes bemessen ist.

6. Medizinisches Elektrodensystem nach Anspruch 1, wobei das erste Bild, das zweite Bild, das dritte Bild und das vierte Bild auf der Grafikseite des jeweiligen Elektrodenpads jeweils derartig orientiert sind, dass der Bildkörper auf den Patienten ausgerichtet ist, wenn das jeweilige Elektrodenpad an der korrekten Position auf dem Patienten angebracht wird.

7. Medizinisches Elektrodensystem nach Anspruch 6, wobei eine lange Dimension des ersten Elektrodenpads zur linken Seite des ersten Bilds und des zweiten Bilds orientiert ist, und wobei weiterhin eine kurze Dimension des zweiten Elektrodenpads zur linken Seite des dritten Bilds orientiert ist, und wobei eine lange Dimension des zweiten Elektrodenpads zur linken Seite des vierten Bilds orientiert ist, wenn das erste, zweite, dritte und vierte Bild in aufrechter Position betrachtet wird.

8. Medizinisches Elektrodensystem nach Anspruch 7, wobei das Ableitungskabel des ersten Elektrodenpads an einer kurzen Kante des ersten Elektrodenpads über dem ersten und dem zweiten Bild angeschlossen ist, wenn es in aufrechter Position betrachtet wird.

9. Medizinisches Elektrodensystem nach Anspruch 1, weiterhin umfassend eine Abziehlasche, die an einem Ende von jedem Elektrodenpad angeordnet ist, und wobei weiterhin jede Abziehlasche eine unterschiedliche Farbe hat.

10. Medizinisches Elektrodensystem nach Anspruch 1, weiterhin umfassend ein Schalengehäuse mit einer Außenfläche und einem Ablöseüberzug auf einer Innenfläche des Gehäuses, wobei die dem Patienten zugewandte Seite des ersten und des zweiten Elektrodenpads jeweils auf dem Ablöseüberzug angebracht ist,
und wobei weiterhin das erste Bild, das zweite Bild, das dritte Bild und das vierte Bild auf der Außenfläche in der gleichen Orientierung wie das jeweilige Bild auf den angebrachten Elektrodenpads angeordnet sind.

11. Externer Defibrillator (400), umfassend:
- eine Hochspannungsenergiequelle (402), die betriebsfähig ist, um Elektrotherapie abzugeben;
- einen Elektrodenkonnektor (404), der elektrisch mit der Hochspannungsenergiequelle verbunden ist;
- eine Steuereinheit (406);
- einen Modusschalter (408), der betriebsfähig ist, um die Steuereinheit von einem Defibrillationsmodus für Erwachsene auf einen Defibrillationsmodus für Kinder umzuschalten;
- eine Elektrodenaufbewahrungsvorrichtung (410, 411) mit einer Ablöseüberzugfläche; und
- ein medizinisches Elektrodensystem (100) nach Anspruch 1;
wobei das erste und das zweite Elektrodenpad erste und zweite universelle Elektrodenpads (110, 210) angeordnet auf der Ablöseüberzugfläche sind;
wobei das elektrisch mit jedem Elektrodenpad verbundene Ableitungskabel (412, 412') weiterhin mit dem Elektrodenkonnektor verbunden ist;
wobei die Grafikseite (116, 216) von jedem Elektrodenpad eine bedruckbare Oberfläche ist; und
wobei das erste und das zweite Bild eine Grafikangabe angeordnet auf der bedruckbaren Oberfläche des ersten Elektrodenpads sind und das dritte und das vierte Bild eine Grafikangabe angeordnet auf der bedruckbaren Oberfläche des zweiten Elektrodenpads sind.

12. Externer Defibrillator nach Anspruch 11,
wobei die Elektrodenaufbewahrungsvorrichtung eines von Folgendem ist:
- ein Klappgehäuse mit einer Ablöseüberzug-Innenfläche, wobei mindestens eine der Grafikangaben auf der Außenfläche des Schalengehäuses wiedergegeben ist; oder
- eine Platte mit der Ablöseüberzugfläche auf beiden Seiten, wobei das erste und das zweite universelle Elektrodenpad einander zugewandt auf gegenüberliegenden Seiten der Platte angeordnet sind.

13. Externer Defibrillator nach Anspruch 11, wobei der Modusschalter als ein Schlüssel vorgesehen ist, der in einen entsprechenden Schlitz in den Defibrillator eingeführt wird.

14. Externer Defibrillator nach Anspruch 11, wobei jede Grafikangabe auf der bedruckbaren Oberfläche in der gleichen Orientierung orientiert ist wie eine beabsichtigte Platzierung der jeweiligen Elektrode auf dem erwachsenen oder pädiatrischen Patienten.

15. Externer Defibrillator nach Anspruch 14, wobei das erste universelle Elektrodenpad zur Anbringung an der Sternumposition an einem erwachsenen Patienten vorgesehen ist, und wobei weiterhin das Ableitungskabel des ersten universellen Elektrodenpads an dem ersten universellen Elektrodenpad an einer Stelle angebracht ist, die von dem Mittelpunkt des Rumpfs des erwachsenen Patienten abgewandt liegt, wenn das erste universelle Elektrodenpad wie beabsichtigt auf dem erwachsenen Patienten platziert ist.

## Revendications

1. Système d'électrode médicale (100) comprenant :
- un premier et un second patins d'électrodes (110, 210) et
- un fil électrique (112, 212), électriquement raccordé à chaque patin d'électrode ;
dans lequel chacun desdits premier et second patins d'électrode comprend un côté orienté vers le patient, apte à être placé sur un patient, et un côté graphique (116, 216) ;
et en outre dans lequel le côté graphique du premier patin d'électrode comprend :
- une première image d'au moins une partie d'un corps humain adulte (120) montrant un placement correct sur un adulte du premier patin d'électrode, et
- une seconde image d'au moins une partie d'un corps humain pédiatrique (124) montrant un placement pédiatrique correct du premier patin d'électrode ;
et en outre dans lequel le côté graphique du second patin d'électrode comprend :
- une troisième image d'au moins une partie d'un corps humain adulte (220) montrant un second placement adulte correct du second patin d'électrode, et
- une quatrième image d'au moins une partie d'un corps humain pédiatrique (224) montrant un second placement pédiatrique correct du second patin d'électrode.

2. Système d'électrode médicale selon la revendication 1, dans lequel chacun parmi le placement adulte correct du premier patin d'électrode dans la première image, le placement pédiatrique correct du premier patin d'électrode dans la seconde image, le second placement adulte correct du second patin d'électrode dans la troisième image, et le second placement pédiatrique correct du second patin d'électrode dans la quatrième image ont une impression distinctive.

3. Système d'électrode médicale selon la revendication 2, dans lequel la première image comprend en outre une image du second placement adulte correct du second patin d'électrode et la troisième image comprend en outre une image du placement adulte correct du premier patin d'électrode.

4. Système d'électrode médicale selon la revendication 1, dans lequel le placement pédiatrique correct du premier patin d'électrode est une position antérieure sur la seconde image, et dans lequel le second placement pédiatrique correct du second patin d'électrode est une position postérieure sur la quatrième image.

5. Système d'électrode médicale selon la revendication 4, dans lequel chacune des images d'au moins une partie du corps humain pédiatrique est dimensionnée proportionnellement à chacune des images d'au moins une partie du corps humain adulte par rapport à la taille effective d'un être humain adulte ou pédiatrique.

6. Système d'électrode médicale selon la revendication 1, dans lequel chacune des première image, seconde image, troisième image et quatrième image est orientée sur le côté graphique du patin d'électrode respectif, de sorte que le corps de l'image soit aligné avec le patient quand le patin d'électrode respectif est collé dans la position correcte sur le patient.

7. Système d'électrode médicale selon la revendication 6, dans lequel une longue dimension du premier patin d'électrode est orientée vers la gauche de la première image et la seconde image et en outre dans lequel une courte dimension du second patin d'électrode est orientée vers la gauche de la troisième image et en outre, dans lequel une longue dimension du second patin d'électrode est orientée vers la gauche de la quatrième image quand chacune des première, seconde, troisième et quatrième images sont visualisées dans une position verticale.

8. Système d'électrode médicale selon la revendication 7, dans lequel le premier fil électrique du patin d'électrode médicale est branché au premier patin d'électrode à un bord de courte dimension au-dessus des première et seconde images, tel que visualisé dans la position verticale.

9. Système d'électrode médicale selon la revendication 1, comprenant en outre une languette disposée à une extrémité de chaque patin d'électrode et en outre dans lequel chaque languette a une couleur distinctive.

10. Système d'électrode médicale selon la revendication 1, comprenant en outre un boîtier en forme de coquille avec une surface extérieure et un revêtement anti-adhésif sur une surface intérieure du boîtier, dans lequel le côté orienté vers le patient de chacun des premier et second patins d'électrodes est fixé au revêtement anti-adhésif,
et en outre dans lequel chacune parmi la première image, la seconde image, la troisième image et la quatrième image sont disposées sur la surface extérieure dans la même orientation que l'image respective sur les patins d'électrodes raccordés.

11. Défibrillateur externe (400) comprenant :
- une source d'énergie haute tension (402) utilisable pour fournir une électrothérapie ;
- un connecteur d'électrode (404) électriquement branché à une source d'énergie haute tension ;
- un système de commande (406) ;
- un commutateur de mode (408) actionnable de façon à commuter le système de commande d'un mode de défibrillation adulte à un mode de défibrillation pédiatrique ;
- un dispositif de stockage d'électrode (410, 411) ayant une surface de revêtement anti-adhésif ; et
- un système d'électrode médicale (100) selon la revendication 1 ;
dans lequel le premier et le second patins d'électrodes sont un premier et un second patins d'électrodes universels (110, 220) disposés sur la surface du revêtement anti-adhésif ;
dans lequel le fil électrique (412, 412'), électriquement branché à chaque patin d'électrode est en outre branché au connecteur d'électrode ;
dans lequel le côté graphique (116, 216) de chaque patin d'électrode est une surface imprimable ; et
dans lequel la première et la seconde images sont une indication graphique disposée sur la surface imprimable du premier patin d'électrode, et la troisième et la quatrième images sont une indication graphique disposée sur la surface imprimable du second patin d'électrode.

12. Défibrillateur externe selon la revendication 11, dans lequel le dispositif de stockage électrique est soit :
- une coquille ayant une surface de revêtement anti-adhésif intérieure, dans laquelle au moins une des indications graphiques est reproduite sur une surface extérieure de ladite coquille ; ou
- une feuille ayant une surface de revêtement anti-adhésif des deux côtés, dans laquelle chacun des premier et second patins d'électrodes universels est disposé selon une relation face à face sur des côtés opposés de la feuille.

13. Défibrillateur externe selon la revendication 11, dans lequel le commutateur de mode est disposé comme une clé qui est insérée dans une fente correspondante dans le défibrillateur.

14. Défibrillateur externe selon la revendication 11, dans lequel chaque indication graphique est orientée sur la surface imprimable selon la même orientation qu'un placement prévu de l'électrode respective sur le patient adulte et pédiatrique.

15. Défibrillateur externe selon la revendication 14, dans lequel le premier patin d'électrode universel est disposé de façon à être fixé au niveau du sternum sur un patient adulte, et en outre dans lequel le fil électrique du premier patin d'électrode universel est branché au premier patin d'électrode universel à un endroit qui est éloigné du centre du torse du patient adulte, quand le premier patin d'électrode universel est placé comme prévu sur le patient adulte.
